Europäisches Patentamt

European Patent Office

Office européen des brevets

(19)

(11) Publication number: 0 234 871
A2

# EUROPEAN PATENT APPLICATION

(12)

(21) Application number: 87301443.5

(22) Date of filing: 19.02.87

(51) Int. Cl.⁴: **C 12 N 15/00**
C 12 N 1/16
//(C12N1/16,C12R1:645)

(30) Priority: 28.02.86 US 835506

(43) Date of publication of application:
02.09.87 Bulletin 87/36

(84) Designated Contracting States:
CH DE FR GB IT Li NL

(71) Applicant: MERCK & CO. INC.
126, East Lincoln Avenue P.O. Box 2000
Rahway New Jersey 07065 (US)

(72) Inventor: Ellis, Ronald W.
1407 Edgevale Road
Overbrook Hills Pennsylvania 19151 (US)

Hofmann, Kathryn J.
511 Bending Lane
King of Prussia Pennsylvania 19406 (US)

Schultz, Loren D.
421 Oak Drive
Harleysville Pennsylvania 19438 (US)

(74) Representative: Hesketh, Alan, Dr.
European Patent Department Merck & Co., Inc. Terlings
Park Eastwick Road
Harlow Essex, CM20 2QR (GB)

(54) Regulatable expression of recombinant proteins in yeast.

(57) A genetic expression system is described which allows regulatable expression of proteins e.g., Epstein-Barr virus membrane antigen, for processing in the secretory pathway. This system is applicable to the expression in yeast of glycoproteins, e.g., Epstein-Barr virus membrane antigen, which exert a toxic effect on the cells or whose expression is negatively selected.

EP 0 234 871 A2

**Description**

## REGULATABLE EXPRESSION OF RECOMBINANT PROTEINS IN YEAST

### BACKGROUND OF THE INVENTION

S. cerevisiae has proven versatile as a host species for the expression of foreign polypeptides. Many different proteins from a variety of species have been expressed in S. cerevisiae, some to levels of over 10% of total cell protein. Typically, expression has been mediated by a plasmid containing yeast regulatory sequences (transcriptional promoter and terminator) circumscribing the structural gene for the expressed polypeptide as well as other sequences required for the selection and amplification of plasmids in both S. cerevisiae and in Escherichia coli.

Alpha mating factor is a sex pheromone of S. cerevisiae which is required for mating between MATα and MATa cells. This tridecapeptide is expressed as a prepropheromone which is directed into the rough endoplasmic reticulum, glycosylated and proteolytically processed to its final mature form which is secreted from cells. This biochemical pathway has been exploited as an expression strategy for foreign polypeptides. The alpha mating factor gene has been molecularly cloned, and its promoter with pre-pro-leader sequence has been utilized to express and secrete a variety of polypeptides. As expected by their traversal of the rough endoplasmic reticulum and Golgi apparatus, foreign proteins can undergo both N- and 0-linked glycosylation events.

S. cerevisiae has 3 genes which encode the enzymes responsible for the utilization of galactose as a carbon source for growth. The GALl, GAL7 and GAL10 genes respectively encode galactokinase, α-D-galactose-l-phosphate uridyltransferase, and uridine diphosphogalactose-4-epimerase. In the absence of galactose, very little expression of these enzymes is detected. If cells initially are grown on glucose and galactose is added to the culture, these three enzymes are induced coordinately when glucose is depleted by at least l,000-fold. This induction has been shown to occur at the level of RNA transcription. The GALl and GAL10 genes have been molecularly cloned and sequenced. The regulatory and promoter sequences to the 5' sides of the respective coding regions have been placed adjacent to the coding regions of the lacZ gene. These experiments have defined those promoter and regulatory sequences which are necessary and sufficient for galactose induction.

In a variety of recombinant microbial expression systems, the synthesis of many different polypeptides has been shown to be deleterious to the host cell. As a consequence, there is selective pressure against the expression of such polypeptides, such that the only cells which accumulate in a scale-up of such a recombinant culture are those which do not express the foreign polypeptide or express so little of the foreign polypeptide that the culture becomes an uneconomical source of that polypeptide.

### OBJECTS OF THE INVENTION

It is an object of the present invention to provide a method for the recombinant plasmid DNA-regulated expression of foreign proteins whose expression is either unstable in or toxic to yeast. Another object is to specify conditions for the scale-up of the growth of recombinant cells transformed by such a recombinant plasmid vector, such that maximal yields of the foreign protein can be attained in large volumes. Another object is to provide a method for the expression of the Epstein-Barr virus membrane antigen (gp350/gp220) in yeast in glycosylated form. These and other objects of the present invention will be apparent from the following description.

### SUMMARY OF THE INVENTION

The expression of the Epstein-Barr virus membrane antigen (gp350/gp220) gene is negatively selected in yeast due to a toxic effect. A genetic expression system is described which allows regulated expression of proteins for processing in the secretory pathway. This system is applicable to the expression in yeast of glycoproteins which exert a toxic effect on the cells or whose expression is negatively selected.

### BRIEF DESCRIPTION OF THE DRAWINGS

Figure l is a schematic diagram illustrating the construction of plasmid pRJl78.

Figure 2 is a schematic diagram illustrating the construction of plasmid pJCl97.

Figure 3 is a schematic diagram illustrating the construction of plasmid pYEBV-l.

Figure 4 is a schematic diagram illustrating the construction of plasmid pGAL10-MFαl.

Figure 5 is a schematic diagram illustrating the construction of plasmid pYEBV-2.

### DETAILED DESCRIPTION OF THE INVENTION

The present invention is directed to a method for applying the galactose-inducible promoters and alpha mating factor pre-pro-leader sequences to the expression of foreign glycoproteins or proteins processed in the secretory pathway whose expression is either unstable in or toxic to yeast. The present invention also is directed to the use of such a vector system to stably express large quantities of the Epstein-Barr virus membrane antigen (EBMA) in glycosylated form in yeast. More specifically, it is directed to conditions for the scale-up to large volumes of a recombinant culture expressing EBMA. It will be obvious to those skilled in the art that genes of either other foreign glycoproteins or proteins processed in the secretory pathway whose expression is toxic or counterselected in yeast can be stably expressed in large volumes by utilizing this vector-host cell system.

The alpha mating factor gene MFαl has been cloned onto a plasmid vector from S. cerevisiae genomic DNA. This DNA is digested with HindIII and self-ligated to form a new plasmid DNA with a unique HindIII site on the 3′ side of the pre-pro-leader sequence. An oligonucleotide adaptor containing translational termination signals in all three reading frames then is inserted into the unique HindIII site in such a fashion that a unique HindIII site is retained on the 5′ side of the oligonucleotide insert. The resulting plasmid is digested with HindIII, made blunt-ended with the Klenow fragment of DNA polymerase I, ligated with BamHI linkers, and self-ligated. This plasmid DNA is digested with EcoRI, made blunt-ended with the Klenow fragment of DNA polymerase I, ligated with BclI linkers, digested with BclI, and the 1.4 kilobase pair (kbp) fragment containing the alpha mating factor gene is isolated. The E. coli - S. cerevisiae shuttle vector pCI/I is digested with BamHI and then ligated with the 1.4 kbp BclI fragment described above. This plasmid DNA (pJCI97) is digested with SalI, made blunt-ended with the Klenow fragment of DNA polymerase I, and digested with PstI for the isolation of a 0.35 kbp fragment. The E. coli S. cerevisiae shuttle vector YEp5I is digested with BclI, made flush-ended with the Klenow fragment of DNA polymerase I, and digested with SalI for the isolation of a 6.3 kbp fragment. These two fragments, together with a 35 bp SalI-PstI synthetic oligonucleotide adapter encoding the ATG and first 8 amino acids of the alpha mating factor pre-pro-leader, are ligated together to create a new expression plasmid (pGALI0-MFαI). The salient features of this vector are: (1) E. coli-derived sequences for the selection and amplification of the plasmid in E. coli, (2) S. cerevisiae-derived sequences for the selection and amplification of the plasmid in S. cerevisiae, (3) yeast GALI0 promoter, (4) yeast alpha mating factor pre-leader for directing the translational product into the rough endoplasmic reticulum, (5) yeast alpha mating factor pro-leader which encodes N-glycosylation signal sequences and which is cleaved by the KEX2 and STEI3 proteases (gene products), (6) a unique BamHI site for the cloning of the gene encoding a foreign polypeptide which is to be expressed, (7) translational termination sequences in all three reading frames, (8) a transcriptional termination sequence.

The EBMA proteins represent 350,000 and 220,000 dalton glycoproteins encoded by the virus. The structural gene for both proteins is identical and is wholly contained in the BamHI-L genomic DNA fragment. The EBMA gene fragment is cloned into the pGALI0-MFαI expression vector. This recombinant plasmid is introduced to S. cerevisiae and transformed clones are selected. Cells are grown in glycerol-lactic acid medium; galactose is added to the cultures to induce expression. Lysates are prepared, electrophoresed in polyacrylamide gels, and Western blotted to nitrocellulose. A 350,000 dalton glycoprotein is found to be specific to EBMA by virtue of its presence only in transformants, its reactivity with human convalescent Epstein-Barr virus sera and a specific monoclonal antibody, and its lack of reactivity with pooled negative human sera.

Previous attempts have been made to express EBMA in an expression vector similar to that described above, except that expression of EBMA is driven from the alpha mating factor promoter which is constitutively transcribed at high levels. In every case where growth of the transformant is attempted in a volume of greater than ten milliliters, expression of EBMA disappears. When the cells are plated out during the course of growth in liquid culture, it is found that there are an increasing number of large colonies equal in size to the nontransformed controls. The large size of the colony reflects a more rapid growth rate than that of a small colony. When Western blots are performed upon the large and small colonies of transformants, the large colonies invariably express no EBMA, while the small colonies invariably do express EBMA. The increasing number of large colonies observed upon plating out during serial growth reflects a negative physiological effect of EBMA upon S. cerevisiae. This negative effect results in selection pressure against expression, such that ultimately no expression of the polypeptide is observed in larger volumes.

These observations highlight the utility of the pGALI0-MFαI vector for the expression of foreign glycoproteins or proteins processed through the secretory pathway whose expression is negatively selected or toxic to S. cerevisiae. It is obvious to those skilled in the art that the regulatable GALI0 promoter, or GALI, GAL2, GAL7, or MELI promoters which function in an indistinguishable manner, enable the growth of a recombinant S. cerevisiae culture to be scaled up to a production-scale volume before synthesis of the recombinant protein is initiated, such that negative effects on the host cell are minimized. Moreover, it is obvious to those skilled in the art that an expression vector containing another promoter, physiologically inducible by means other than galactose, can be utilized to direct expression of foreign proteins linked to the alpha mating factor pre-pro-leader. Furthermore, it is obvious to those skilled in the art that an expression vector containing a pre- or pre-pro-leader other than that of alpha mating factor can be utilized for linkage to a foreign protein whose expression is driven by a regulatable promoter. It is obvious to those skilled in the art that a suitable assay system, e.g., Western blot or radioimmunoassay, should be utilized to assay expression of a foreign glycoprotein in this system in order to optimize the time of harvesting of the culture for attaining a maximal yield.

The genus Saccharomyces is composed of a variety of species. Most commonly used is Saccharomyces cerevisiae, or baker's yeast, as a host for the recombinant DNA-mediated expression of a variety of foreign polypeptides. However, the distinctions among other species of the genus Saccharomyces are not always well-defined. Many of these species are capable of interbreeding with S. cerevisiae and are likely to possess regulatable promoters, such as GALI0, analogous or identical to those in S. cerevisiae. Therefore, it will be obvious to those skilled in the art that, for the regulatable expression of foreign glycoproteins or proteins processed through the secretory pathway whose expression is negatively regulated or toxic to S. cerevisiae, the selection of a host strain extends to other species of the genus Saccharomyces, including but not limited to carlsbergensis, uvarum, rouxii, montanus, Kluyveri, elongisporus, norbensis, oviformis, and diastaticus.

Several yeast genera, such as Hansenula, Candida, Torulopsis, and Pichia, have been shown to contain similar metabolic pathways for the utilization of methanol as a sole carbon source for growth. The gene for alcohol oxidase, an enzyme which participates in this metabolic pathway, has been isolated from Pichia pastoris. The P. pastoris alcohol oxidase promoter has been isolated and shown to be sensitive to methanol induction of expression. This promoter has been placed on an expression vector, where it has been shown to promote the expression of foreign genes cloned adjacent to it. Such an inducible promoter is useful for the expression in yeast of peptides or polypeptides, such as EBMA, whose expression is negatively selected. The pre-pro-leader of alpha mating factor is but one example of a leader sequence in S. cerevisiae. Such leader sequences, which direct primary translational products into the rough endoplasmic reticulum for traversal of the glycosylation pathway, have been found in all eukaryotic cells studied . It is by virtue of the presence of a pre-leader sequence that the translational product of the EBMA gene is glycosylated in virally-infected cells and in recombinant yeast. The pro-leader is not required for directing primary translational products into the rough endoplasmic reticulum. As is obvious to those skilled in the art, this sequence can be deleted with the use of appropriate restriction endonucleases, and/or oligonucleotide synthesis of the pre-leader sequence alone. Therefore, it will be obvious to those skilled in the art that the concepts of using a suitable promoter and pre-leader sequence for the expression and glycoylation of EBMA extend the range of suitable yeast hosts to species of yeast from the Families Saccharomycetaceae and Cryptococcaceae, including but not limited to species from the genera Pichia, Candida, Hansenula, Torulopsis, Kluyveromyces, and Saccharomycopsis.

The following examples illustrate the present invention without, however, limiting the same thereto. The disclosure of each reference mentioned in the following examples is hereby incorporated by reference.

## EXAMPLE I

Preparation of an alpha mating factor expression vector

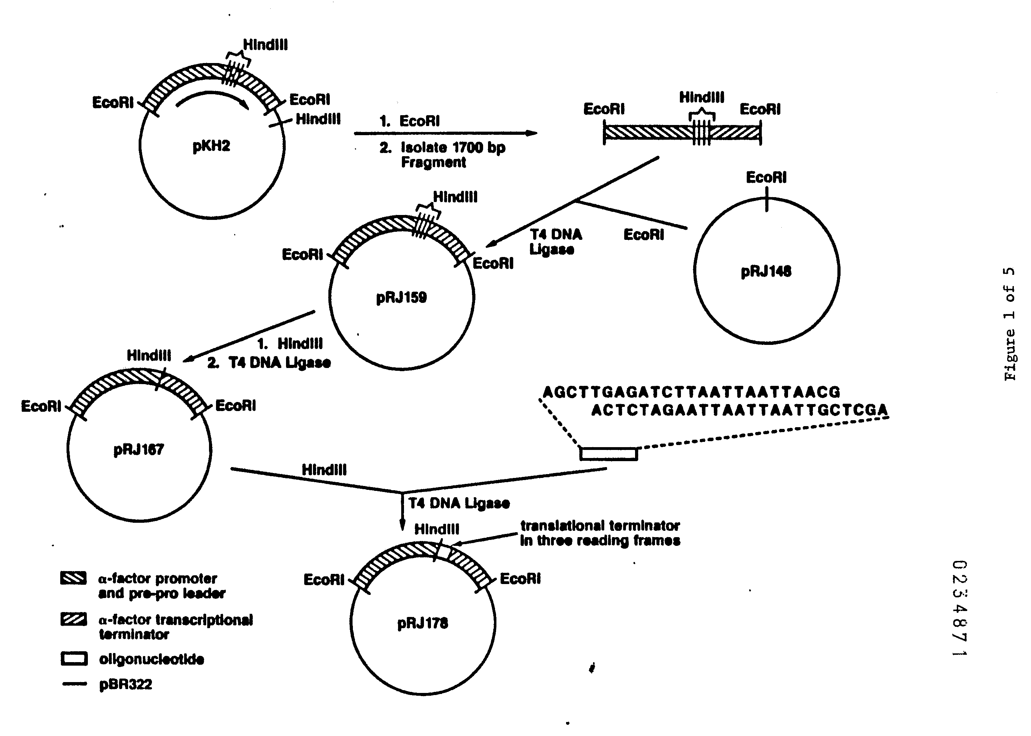

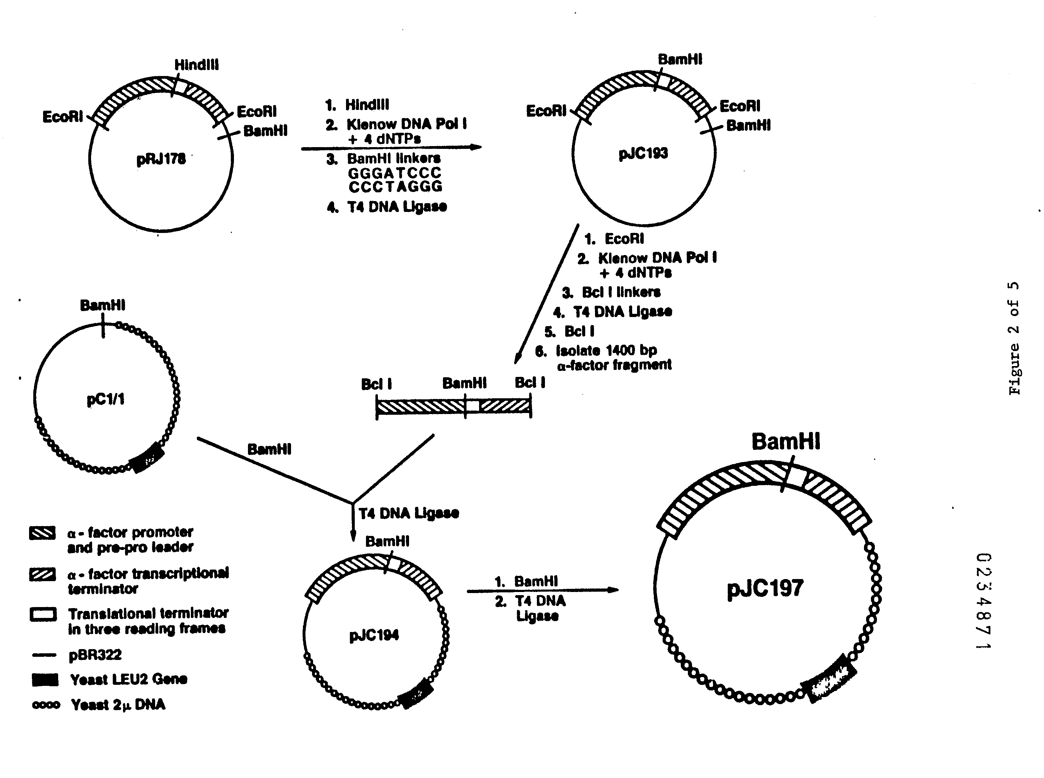

Plasmid pKH2 with the alpha mating factor gene MFαl cloned onto a plasmid vector from S. cerevisiae genomic DNA was prepared following the procedures of Kurjan et al. [Cell 30: 933 (1982)] and Singh et al. [Nucleic Acids Res. II: 4049 (1983)]. The resulting plasmid pKH2 was digested with EcoRI, and the 1.7 kbp fragment bearing the alpha mating factor gene was purified by preparative agarose gel electrophoresis (Figure I, top). Plasmid pRJI48 (a modified pBR322 lacking the HindIII site) was digested with EcoRI and ligated with the 1.7 kbp fragment to yield the plasmid pRJI59 (Figure I,

middle). This DNA was digested with HindIII and self-ligated to form plasmid pRJI67, which now has a unique HindIII site (Figure I, bottom). Plasmid pRJI67 was digested with HindIII and modified by the insertion of a synthetic oligonucleotide adaptor to yield a new plasmid (pRJI78) containing a unique HindIII site which is to the 3' side of the promoter and pre-pro-leader and to the 5' side of the translational termination signals in all three reading frames (Figure I, bottom). The HindIII site was converted to a BamHI site by digestion with HindIII, flush-ending with the Klenow fragment of DNA polymerase I, addition of BamHI linkers and self-ligation to form plasmid pJCI93 (Figure 2, top). This plasmid was digested with EcoRI, flush-ended with the Klenow fragment of DNA polymerase I, modified by the addition of BclI linkers, digested with BclI, and the 1.4 kbp fragment bearing the alpha mating factor gene isolated by preparative agarose gel electrophoresis (Figure 2, middle). This resulting BclI fragment then was inserted into the unique BamHI site of pCI/I, destroying the original BamHI site in the process (plasmid pJCI94; Figure 2, bottom). This DNA was digested with BamHI and self-ligated to remove excess BamHI linkers (Figure 2, bottom). As a result, a new alpha mating factor expression plasmid (pJCI97) was created which has the following salient features:

(I) E. coli derived sequences for the selection (bla gene) and amplification (ori) of the plasmid in E. coli, (2) S. cerevisiae-derived sequences for the selection (LEU2) and amplification (2-micron DNA) of the plasmid in S. cerevisiae, (3) yeast alpha mating factor promoter, (4) yeast alpha mating factor pre-leader for directing the translational product into the rough endoplasmic reticulum, (5) yeast alpha mating factor pro-leader which encodes N-glycosylation signal sequences and which is cleaved by the KEX2 and STEI3 proteases (gene products) [Julius et al., Cell 32: 839 (1983); Julius et al., Cell 37: 1075 (1984)], (6) a unique BamHI site for the cloning of a foreign polypeptide to be expressed, (7) a translational termination sequence in all three reading frames, (8) a transcriptional termination sequence.

## EXAMPLE 2

Cloning and expression of the EBMA gene in the alpha mating factor expression vector pJCI97

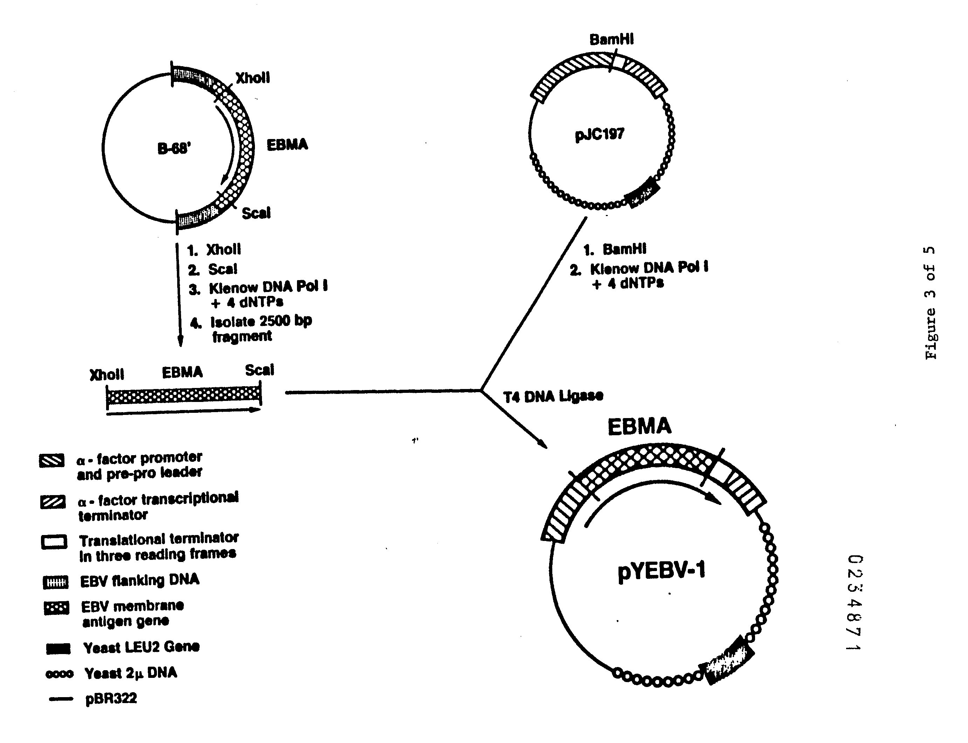

The EBMA proteins represent 350,000 dalton and 220,000 dalton glycoproteins encoded by the virus [Qualtiere et al., Virology 102: 360 (1980)]. The structural gene for both proteins is identical and is wholly contained in the BamHI-L genomic DNA fragment [Hummel et al., J. Virol. 49: 413 (1984)]. The B-68' plasmid carrying the BamHI-L fragment was digested with XhoII and ScaI, and the 2.5 kbp fragment containing the EBMA structural gene sequence was made flush-ended with the Klenow fragment of DNA polymerase I and purified by preparative agarose gel electrophoresis (Figure 3, top). The alpha mating factor vector pJCI97 was digested with BamHI and made flush-ended with the Klenow fragment of DNA polymerase I. The EBMA fragment was blunt-end ligated to the vector (Figure 3, bottom). This recombinant plasmid (pYEBV-I) was introduced to S. cerevisiae strain IH868 [also called DC6 (MATα, leu2, his4, canI, gal2), Kurjan et al., ibid.], and transformed clones were selected and amplified. Lysates were prepared, electrophoresed in polyacrylamide gels and Western blotted to nitrocellulose. Both 175,000 and 350,000 dalton glycoproteins were found to be specific to EBMA by virtue of their presence only in transformants, their reactivity with human convalescent Epstein-Barr virus sera and an EBMA-specific monoclonal antibody [Qualtiere et al., Proc. Natl. Acad. Sci. USA 79: 6I6 (1982)], and their lack of reactivity with pooled negative human sera. In every case where growth of the pYEBV-I transformant was attempted in a volume of greater than ten milliliters, expression of EBMA was not detected. While initially the culture yielded essentially only small colonies when plated on selective media, it was found that there was an increasing relative number of large colonies equal in size to the nontransformed controls when the cells were plated out during the course of growth in liquid culture. The large size of the colony reflected a more rapid growth rate than that of a small colony. When Western blots were performed upon the large and small colonies of transformants, the large colonies invariably expressed no EBMA, while the small colonies invariably did express EBMA. The increasing number of large colonies observed upon plating out during serial growth reflected a negative physiological effect of EBMA upon S. cerevisiae. This negative effect results in selection pressure against expression, such that ultimately no expression of the polypeptide is observed in volumes greater than ten milliliters.

EXAMPLE 3

Preparation of a regulatable expression vector, pGALI0-MFαI

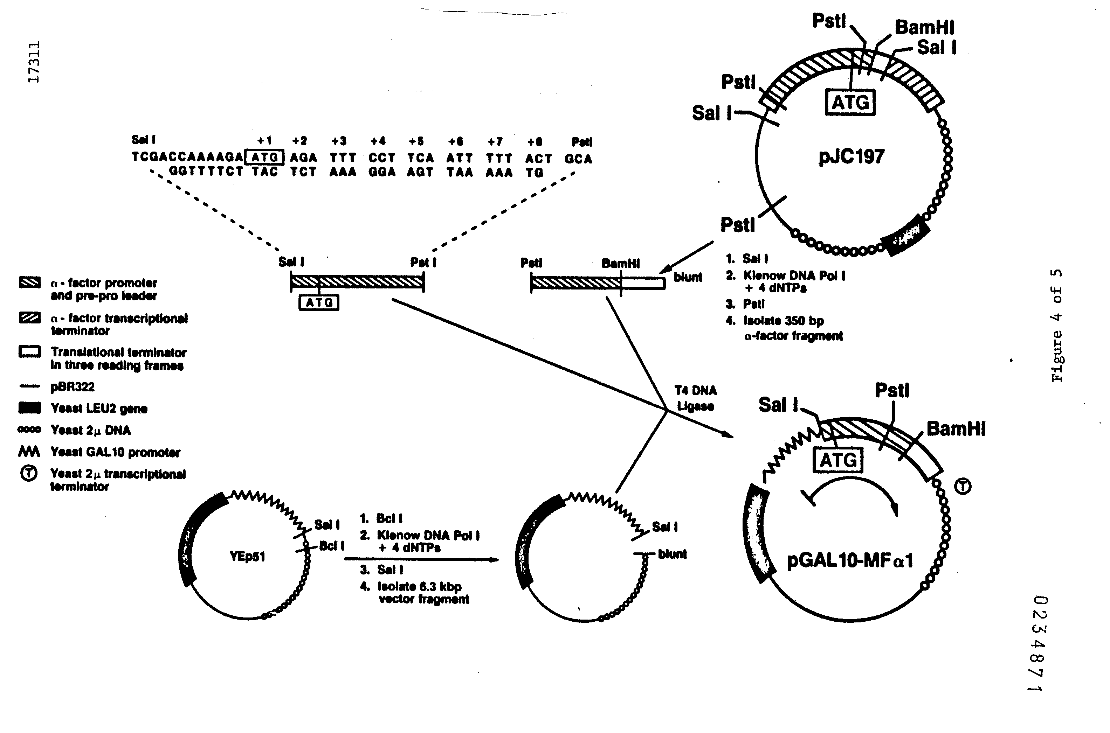

The alpha mating factor expression vector pJCI97 was digested with SaII, made blunt-ended with the Klenow fragment of DNA polymerase I and digested with PstI. A 0.35 kbp fragment was isolated by preparative agarose gel electrophoresis; this fragment contained most of the alpha mating factor pre-pro-leader sequences, a BamHI cloning site, and translational termination signals in all three reading frames (Figure 4, top). The E. coli - S. cerevisiae shuttle vector YEp5I [Broach et al., p. 83 in Experimental Manipulation of Gene Expression, Academic Press, New York (1983)] contains the yeast GALI0 gene promoter sequences. This DNA was digested with BcII, made flush-ended with the Klenow fragment of DNA polymerase I and digested with SaII. A 6.3 kbp fragment was isolated by preparative gel electrophoresis (Figure 4, top). A 35 bp SaII - PstI synthetic oligonucleotide adapter was synthesized, having the structure

TCGACCAAAAGAATGAGATTTCCTTCAATTTT-
TACTGCA        GGTTTTCTTACTCTAAAGGAAGT-
TAAAAATG.

This adapter contains II base pairs of the alpha mating factor gene non-translated leader and the ATG and first 8 amino acids of the alpha mating factor pre-pro-leader (Figure 4, top). The 6.3 kbp, 0.35 kbp and 35 bp fragments were ligated together to create a new expression plasmid, pGALI0-MFαI (Figure 4, bottom). The salient features of this vector are: (I) E. coli-derived sequences for the selection (bla) and amplification (ori) of the plasmid in E. coli, (2) S. cerevisiae-derived sequences for the selection (LEU2) and amplification (2-micron DNA) of the plasmid in S. cerevisiae, (3) yeast GALI0 promoter, (4) yeast alpha mating factor pre-leader for directing the translational product into the rough endoplasmic reticulum, (5) yeast alpha mating factor pro-leader which encodes N-glycosylation signal sequences and which is cleaved by the KEX2 and STEI3 proteases (gene products) [Julius et al., ibid. (1983); Julius et al., ibid. (1984)], (6) a unique BamHI site for the cloning of a foreign polypeptide to be expressed, (7) a translational termination sequence in all three reading frames, and (8) a transcriptional termination sequence.

EXAMPLE 4

Cloning and expression of the EBMA gene in the regulatable expression vector, pGALI0-MFαI

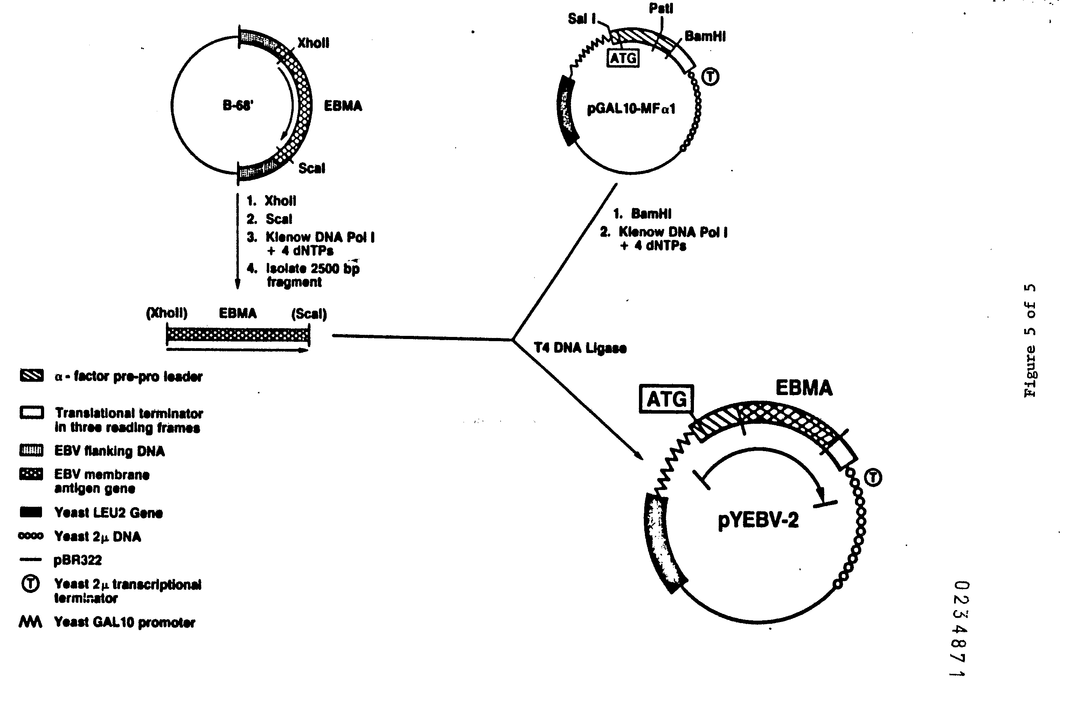

The B-68' plasmid carrying the BamHI-L DNA fragment containing the EBMA gene was digested with XhoII and ScaI, and the 2.5 kbp fragment containing the EBMA structural gene was made flush-ended with the Klenow fragment of DNA polymerase I (Figure 5, top). The expression vector pGALI0-MFαI was digested with BamHI and made flush-ended with the Klenow fragment of DNA polymerase I. The 2.5 kbp EBMA fragment was ligated to the vector. This recombinant plasmid pYEBV-2 (Figure 5, bottom) was introduced to S. cerevisiae strain DC04 (MATa, leu2-04, adeI) [Broach et al., Cell 2I: 50I (1980)], and transformed clones were selected and amplified. Cells were grown in glycerol-lactic acid to a I0 liter volume, during which time there was no detectable synthesis of EBMA by Western blot. When the culture had reached early exponential phase, galactose was added to a final concentration of 2% (w/v). Synthesis of EBMA, as diagnosed by Western blots of lysates resolved by polyacrylamide gel electrophoresis, was first detectable within 4 hours after galactose addition, was maximal, at 48 hours and was diminished at 96 hours.

**Claims**

I. A plasmid expression vector containing an E. coli-derived DNA sequence for selection and propagation in E. coli, a yeast-derived DNA sequence for selection and propagation of the plasmid in a species of the Families Cryptococcaceae and Saccharomycetaceae, a yeast regulatable promoter DNA sequence, a pre- or pre-pro-leader sequence, a translational termination sequence for at least one reading frame, a yeast transcriptional termination sequence, and a unique restriction endonuclease cloning site for insertion of a sequence of DNA coding for a predetermined peptide or polypeptide sequence that is deleterious to the host when expressed in a host cell.

2. A plasmid expression vector according to Claim I wherein the predetermined peptide or polypeptide sequence is deleterious when expressed in a host cell which is a species of the Families Saccharomycetaceae or Cryptococcaceae.

3. A plasmid expression vector according to Claim 2 having a sequence of DNA that codes for the Epstein-Barr virus membrane antigen (gp 350/gp 220).

4. A plasmid expression vector according to Claim 2 wherein the pre-leader or the pre-proleader sequence is derived from the alpha mating factor gene.

5. A species of the Families Saccharomycetaceae or Cryptococcaceae transformed by a plasmid expression vector according to Claim 2.

6. A process for obtaining a predetermined peptide or polypeptide which is deleterious when expressed in a host that is a species of the Families Saccharomycetaceae or Cryptococcaceae which comprises: (1) transforming cells of a species of said Families with a plasmid according to Claim 2, (2) culturing the transformed cells and (3) recovering the protein from the cultured cells.

7. A process according to Claim 6 wherein the predetermined peptide or polypeptide is Epstein-Barr virus membrane antigen (gp 350/gp 220).

8. A process according to either Claim 6 or Claim 7 wherein the species is of the genus Saccharomyces.

9. A process according to either Claim 6 or Claim 7 wherein the species is S. cerevisiae.

10. A process according to Claim 7 wherein the plasmid directs expression of the Epstein-Barr virus membrane antigen in glycosylated form.

HindIII

EcoRI

EcoRI
HindIII

**pKH2**

1. EcoRI

2. Isolate 1700 bp Fragment

EcoRI

HindIII

EcoRI

EcoRI

**pRJ148**

HindIII

EcoRI

EcoRI

T4 DNA Ligase

EcoRI

**pRJ159**

1. HindIII
2. T4 DNA Ligase

HindIII

EcoRI

EcoRI

**pRJ167**

AGCTTGAGATCTTAATTAATTAACG
ACTCTAGAATTAATTAATTGCTCGA

HindIII

T4 DNA Ligase

HindIII

translational terminator in three reading frames

EcoRI

EcoRI

**pRJ176**

α-factor promoter and pre-pro leader

α-factor transcriptional terminator

oligonucleotide

pBR322

Figure 1 of 5

0234871

α - factor promoter
and pre-pro leader

α - factor transcriptional
terminator

Translational terminator
In three reading frames

pBR322

Yeast LEU2 Gene

Yeast 2μ DNA

pC1/1

BamHI

pRJ178

EcoRI

HindIII

EcoRI
BamHI

1. HindIII
2. Klenow DNA Pol I
   + 4 dNTPs
3. BamHI linkers
   GGGATCCC
   CCCTAGGG
4. T4 DNA Ligase

BamHI

pJC194

BamHI

T4 DNA Ligase

Bcl I

BamHI

Bcl I

1. EcoRI
2. Klenow DNA Pol I
   + 4 dNTPs
3. Bcl I linkers
4. T4 DNA Ligase
5. Bcl I
6. Isolate 1400 bp
   α-factor fragment

pJC193

EcoRI

BamHI

EcoRI
BamHI

1. BamHI
2. T4 DNA
   Ligase

pJC197

BamHI

Figure 2 of 5

Figure 3 of 5

Legend:
- α - factor promoter and pre-pro leader
- α - factor transcriptional terminator
- Translational terminator in three reading frames
- pBR322
- Yeast LEU2 gene
- Yeast 2μ DNA
- Yeast GAL10 promoter
- Yeast 2μ transcriptional terminator

Sal I
TCGACCAAAGA ATG AGA TTT CCT TCA ATT TTT ACT GCA
AGTTTTCT TAC TCT AAA GGA AGT TAA AAA TG
+1 +2 +3 +4 +5 +6 +7 +8 Pst I

ATG
Sal I
Pst I

YEp51
Sal I
Bcl I

1. Bcl I
2. Klenow DNA Pol I + 4 dNTPs
3. Sal I
4. Isolate 6.3 kbp vector fragment

Sal I
blunt

T4 DNA Ligase

Pst I
BamHI
blunt

1. Sal I
2. Klenow DNA Pol I + 4 dNTPs
3. Pst I
4. Isolate 350 bp α-factor fragment

Sal I
Pst I
pJC197
ATG
Pst I
BamHI
Sal I

pGAL10-MFα1
ATG
Sal I
Pst I
BamHI

Figure 4 of 5

0234871

Figure 5 of 5